# EUROPEAN PATENT APPLICATION

(11) **EP 3 428 650 A2**
(43) Date of publication of application: **16.01.2019**
(21) Application number: 18183279.1
(22) Date of filing: 12.07.2018
(51) Int. Cl.: G01N 33/68

(54) **METHOD FOR QUANTIFYING COGNITIVE DYSFUNCTION DISEASE BIOMARKER USING MASS SPECTROMETRY AND MASS SPECTROMETER**

(30) Priority: 14.07.2017 JP 2017137725
(71) Applicant: Shimadzu Corporation, Kyoto 604-8511 (JP)
(72) Inventor: MATSUBARA, Toshiya, Kyoto-shi,, Kyoto 604-8511 (JP); KAWAKAMI, Daisuke, Kyoto-shi,, Kyoto 604-8511 (JP)
(74) Representative: Webster, Jeremy Mark

(57) **Abstract**

In the present invention, multiple reaction monitoring (MRM) measurements of at least one peptide selected from the group consisting of 14 peptides as biomarkers of cognitive dysfunction diseases contained in a biological sample are performed using a mass spectrometer capable of MS/MS measurement, and the peptide is quantified based on a result thereof. In this case, for each peptide, an MRM transition which is a combination of a mass-to-charge ratio of a precursor ion and a mass-to-charge ratio of a product ion is stored in advance in a storage unit of the mass spectrometer as a measurement condition of the MRM measurement, and the MRM transition is used when cognitive dysfunction disease biomarkers are detected.

SEQUENCE LISTING

<110>    Shimadzu Corporation

<120>   Quantification method of biomarkers for detecting cognitive dysfunction disease
using mass spectrometry

<130>   K1170182

<160>   14

<170>   PatentIn version 3.5

<210>   1
<211>   17
<212>   PRT
<213>   Homo sapiens

<400>   1

Ala Pro Leu Gln Pro Val Thr Pro Leu Gln Leu Phe Glu Gly Arg Arg
1               5                   10                  15

Asn

<210> 2

<211> 16

<212> PRT

<213> Homo sapiens

<400> 2

Pro Gly Arg Gln Ser Gln Glu Gly Ala Gly Leu Pro Ser His His Gly

1               5                   10                  15

<210> 3

<211> 11

<212> PRT

<213> Homo sapiens

<400> 3

Ala Ala Pro Pro Gly Ala Glu Gly Asn Arg Thr

1               5                   10

<210> 4

<211> 17

<212> PRT

<213> Homo sapiens

<400> 4

Lys Leu Ala Glu Leu Pro Ala Ala Ala Gln Pro Ser Ala Glu Asp Ser
1               5                   10                  15

Asp

<210> 5
<211> 21
<212> PRT
<213> Homo sapiens

<400> 5

Thr Ala Thr Ser Glu Tyr Gln Thr Phe Phe Asn Pro Arg Thr Phe Gly
1               5                   10                  15

Ser Gly Glu Ala Asp
            20

<210> 6

<211> 14

<212> PRT

<213> Homo sapiens

<400> 6

Ala Pro Val Ile His Gln Glu Met Ile Gly Gly Leu Arg Asn

1               5                   10

<210> 7

<211> 16

<212> PRT

<213> Homo sapiens

<400> 7

Gln Thr Ala Ser Gly Gln Ala Leu Lys Gly Asp Gly Gly Leu Tyr Ser

1               5                   10                  15

<210> 8

<211> 20

<212> PRT

<213> Homo sapiens

<400> 8

Gly Leu Gly Leu Ser Tyr Leu Ser Ser His Ile Ala Asn Val Glu Arg

1               5                   10                  15

Val Pro Phe Asp

            20

<210> 9

<211> 14

<212> PRT

<213> Homo sapiens

<400> 9

Arg Ser Gly Gly Asn Ala Thr Leu Gln Val Asp Ser Trp Pro

1               5                   10

<210> 10

<211> 19

<212> PRT

<213> Homo sapiens

<400> 10

Asn Ile Ala Ile Val Gly Asp Val Arg Leu Val Gly Glu Val Pro Ser

1               5                   10                  15

Ser Gly Thr

<210> 11

<211> 13

<212> PRT

<213> Homo sapiens

<400> 11

Thr Ala Thr Ser Glu Tyr Gln Thr Phe Phe Asn Pro Arg

1               5                   10

<210> 12

<211> 12

<212> PRT

<213> Homo sapiens

<400> 12

Gly Leu Asp Glu Asp Ser Asp Arg Ala Ile Glu Gly

1               5                   10

<210> 13

<211> 13

<212> PRT

<213> Homo sapiens

<400> 13

Gly Leu Asp Glu Asp Ser Asp Arg Ala Ile Glu Gly Arg

1               5                   10

<210> 14

<211> 14

<212> PRT

<213> Homo sapiens

<400> 14

Gly Val Ser Gly Ser His Phe Val Gly Val Asp Gly Val Arg

1               5                   10

SEQUENCE LISTING

<110>  Shimadzu Corporation

<120>  Quantification method of biomarkers for detecting cognitive
dysfunction disease using mass spectrometry

<130>  K1170182

<160>  14

<170>  PatentIn version 3.5

<210>  1
<211>  17
<212>  PRT
<213>  Homo sapiens

<400>  1

Ala Pro Leu Gln Pro Val Thr Pro Leu Gln Leu Phe Glu Gly Arg Arg
1               5                   10                  15

Asn

<210>  2
<211>  16
<212>  PRT
<213>  Homo sapiens

<400>  2

Pro Gly Arg Gln Ser Gln Glu Gly Ala Gly Leu Pro Ser His His Gly
1               5                   10                  15

<210>  3
<211>  11
<212>  PRT
<213>  Homo sapiens

<400>  3

Ala Ala Pro Pro Gly Ala Glu Gly Asn Arg Thr
1               5                   10

<210>  4
<211>  17
<212>  PRT
<213>  Homo sapiens

<400>  4

Lys Leu Ala Glu Leu Pro Ala Ala Ala Gln Pro Ser Ala Glu Asp Ser
1               5                   10                  15

Asp

<210>  5
<211>  21
<212>  PRT
<213>  Homo sapiens

<400>  5

Thr Ala Thr Ser Glu Tyr Gln Thr Phe Phe Asn Pro Arg Thr Phe Gly
1               5                   10                  15

Ser Gly Glu Ala Asp
            20

<210>  6
<211>  14
<212>  PRT
<213>  Homo sapiens

<400>  6

Ala Pro Val Ile His Gln Glu Met Ile Gly Gly Leu Arg Asn
1               5                   10

<210>  7
<211>  16
<212>  PRT
<213>  Homo sapiens

<400>  7

Gln Thr Ala Ser Gly Gln Ala Leu Lys Gly Asp Gly Gly Leu Tyr Ser
1               5                   10                  15

<210>  8
<211>  20
<212>  PRT
<213>  Homo sapiens

<400>  8

Gly Leu Gly Leu Ser Tyr Leu Ser Ser His Ile Ala Asn Val Glu Arg
1               5                   10                  15

Val Pro Phe Asp
            20

<210>  9
<211>  14
<212>  PRT
<213>  Homo sapiens

<400>  9

Arg Ser Gly Gly Asn Ala Thr Leu Gln Val Asp Ser Trp Pro
1               5                   10

<210>  10
<211>  19
<212>  PRT
<213>  Homo sapiens

<400>  10

Asn Ile Ala Ile Val Gly Asp Val Arg Leu Val Gly Glu Val Pro Ser
1               5                   10                  15

Ser Gly Thr

<210>  11
<211>  13
<212>  PRT
<213>  Homo sapiens

<400>  11

Thr Ala Thr Ser Glu Tyr Gln Thr Phe Phe Asn Pro Arg
1               5                   10

<210>  12
<211>  12
<212>  PRT
<213>  Homo sapiens

<400>  12

Gly Leu Asp Glu Asp Ser Asp Arg Ala Ile Glu Gly
1               5                   10

<210>  13
<211>  13
<212>  PRT
<213>  Homo sapiens

<400>  13

Gly Leu Asp Glu Asp Ser Asp Arg Ala Ile Glu Gly Arg
1               5                   10

<210>  14
<211>  14
<212>  PRT
<213>  Homo sapiens

<400>  14

Gly Val Ser Gly Ser His Phe Val Gly Val Asp Gly Val Arg
1               5                   10

## Description

### TECHNICAL FIELD

The present invention relates to a method for detecting and quantifying biomarkers of cognitive dysfunction diseases by quantifying a plurality of types of peptides using mass spectrometry and a mass spectrometer.

### BACKGROUND ART

Cognitive dysfunction diseases including Alzheimer's disease are diseases that cause a decline in memory ability and cognitive ability of a patient, and the disease progresses gradually and continuously over 10 to 20 years. In an advanced cognitive dysfunction disease in which the disease has progressed, not only the loss of memory function and cognitive function, but also the collapse of personality is caused and the social life function of the patient is lost and so development of an effective therapeutic agent is desired.

In Japan, several therapeutic agents have been developed and used, including donepezil hydrochloride as an acetylcholinesterase inhibitor approved as a therapeutic agent for Alzheimer type dementia in 1999, but all therapeutic agents only slow the progression of the symptoms and does not restore the declined function. Therefore, in order to make the current therapeutic agents more effective, it is desirable to find a cognitive dysfunction disease at an early stage.

The following three methods are mainly used for diagnosing cognitive dysfunction diseases:

### (1) Diagnosis by interview

This is a method in which a specialized clinician inquires about the orientation (current year and month, time, the basic situation such as where you are), memory ability, computational power, and language ability of a testee and makes a diagnosis based on the results. Mini-Mental State Examination (MMSE), Hasegawa Dementia Scale-Revised (HDS-R), Wechsler Adult Intelligence Scale, Third Edition (WAIS-III) are available.

### (2) Diagnostic imaging

This is a method of measuring the cerebral blood flow rate and glucose metabolism by Positron Emission Tomography (PET) or Single Photon Emission Computed Tomography (SPECT) to make a diagnosis based on the results.

### (3) Biomarker test

This is a method of measuring the quantity and quantity ratio of total tau (t-tau) and amyloid β peptide in cerebrospinal fluid to make a diagnosis based on the results.

The method (1) is usually used for the purpose of screening for cognitive dysfunction disease and does not lead to definitive diagnosis. In addition, patients with cognitive dysfunction diseases classified as serious cannot be diagnosed by HSD-R and MMSE (Non Patent Literature 1)" Nagai Shoten Co., Ltd., April 2004). The method (2) requires the use of special and expensive equipment and it is difficult to implement the method in all medical institutions. Further, in the methods (1) and (2), the diagnostic result may differ depending on the physician in charge, resulting in lack of objectivity.

Although the method (3) has high diagnostic sensitivity and enables objective diagnosis, sampling of cerebrospinal fluid is technically difficult and is not practical.

On the other hand, a difference in the content of a plurality of specific proteins or peptides derived from the proteins contained in blood (including serum and plasma), which is a biological sample relatively easily obtainable, was found between patients having a cognitive dysfunction disease (cognitive dysfunction disease subjects) and those who do not suffer from a cognitive dysfunction disease (non-cognitive dysfunction disease subjects), and a method of detecting cognitive dysfunction diseases using these proteins or peptides as biomarkers has been discussed (Patent Literatures 1 to 6). Among these biomarkers, some biomarkers show a difference in content not only between subjects with cognitive dysfunction disease and non-cognitive dysfunction disease subjects, but also between subjects with cognitive dysfunction diseases of different degrees of progression, and thus, the measurement of content of such biomarkers is expected as an effective method for early diagnosis of cognitive dysfunction diseases and determination of the degree of progression of symptoms of the disease.

### CITATION LIST

### PATENT LITERATURE

Patent Literature 1: JP 2010-271078 A
Patent Literature 2: JP 2012-037349 A
Patent Literature 3: JP 2012-132808 A
Patent Literature 4: JP 2016-028243 A
Patent Literature 5: JP 2016-028244 A
Patent Literature 6: WO 2014/207888

### NON PATENT LITERATURE

Non Patent Literature 1: Edited by Imaharu Nakano and Hidehiro Mizusawa "Yoku wakaru arutsuhaimah byo-jissaini kakawaru hitono tameni (Alzheimer's Disease Understood Well-For Actual Participants)"

### SUMMARY OF INVENTION

### TECHNICAL PROBLEM

In order to achieve an early diagnosis of cognitive dysfunction diseases or to improve the accuracy of determination of the degree of progression, it is necessary to specifically and highly sensitively detect a plurality of types of biomarkers contained in a biological sample. In addition, as the number of patients with cognitive dysfunction diseases is expected to increase along with aging, considering that the number of specimens (the number of biological samples) for diagnosing cognitive dysfunction disease increases, it is necessary to make the time needed for detecting one type of biomarker as short as possible.

In Patent Literatures 1 to 6 described above, as a method for detecting a cognitive dysfunction disease biomarker, a measuring method using an antigen-antibody reaction, an enzyme activity measuring method using a reaction between a substrate and an enzyme, or a measuring method using mass spectrometry is disclosed. However, the measuring method using the antigen-antibody reaction and the enzyme activity measuring method are methods for measuring one type of protein or one type of peptide alone, and it takes time and effort to measure various types of biomarkers. On the other hand, in the measuring method using the mass spectrometry, while it is possible to measure a plurality of types of biomarkers at a time, measurement conditions need to be set appropriately for each of the plurality of types of biomarkers in order to enhance the detection sensitivity, but measurement conditions are not discussed in Patent Literatures 1 to 6. Under present circumstances, as described above, there is no established method for detecting biomarkers of cognitive dysfunction diseases satisfying the above-mentioned desires.

A subject to be solved by the present invention is to provide a technology for detecting a biomarker of cognitive dysfunction disease in a biological sample in a short time specifically with high sensitivity.

### SOLUTION TO PROBLEM

Thus, the present invention aimed at solving the aforementioned problem provides a method for quantifying a cognitive dysfunction disease biomarker based on a result obtained by performing a multiple reaction monitoring (MRM) measurement using a mass spectrometer capable of MS/MS measurement of the cognitive dysfunction disease biomarker contained in a biological sample,
wherein
the cognitive dysfunction disease biomarker includes at least one peptide selected from the group consisting of:
a complement C4-A-derived peptide AD1008 composed of an amino acid sequence represented by SEQ ID NO: 1,
a transcription factor AP-2γ-derived peptide AD1025 composed of an amino acid sequence represented by SEQ ID NO: 2,
an oxytocin receptor-derived peptide AD1042 composed of an amino acid sequence represented by SEQ ID NO: 3,
an E3 ubiquitin ligase HERC-derived peptide AD1046 composed of an amino acid sequence represented by SEQ ID NO: 4,
a prothrombin-derived peptide AD1048 composed of an amino acid sequence represented by SEQ ID NO: 5,
a complement C4-derived peptide AD1049 composed of an amino acid sequence represented by SEQ ID NO: 6,
a tumor necrosis factor receptor superfamily member 16-derived peptide ADPEP109315 composed of an amino acid sequence represented by SEQ ID NO: 7,
a gelsolin-derived peptide ADPEP421488 composed of an amino acid sequence represented by SEQ ID NO: 8,
a neurexin-2-β precursor-derived peptide ADPEP 1315 composed of an amino acid sequence represented by SEQ ID NO: 9,
a neurexin-1-β-derived peptide ADPEP12neu composed of an amino acid sequence represented by SEQ ID NO: 10,
a prothrombin precursor-derived peptide ADPEP1396 composed of an amino acid sequence represented by SEQ ID NO: 11,
a prothrombin precursor-derived peptide ADPEP1039 composed of an amino acid sequence represented by SEQ ID NO: 12,
a prothrombin precursor-derived peptide ADPEP1250 composed of an amino acid sequence represented by SEQ ID NO: 13, and
a protocadherin γ-derived peptide AD1089 composed of an amino acid sequence represented by SEQ ID NO: 14, and
an MRM transition which is a combination of a mass-to-charge ratio of a precursor ion and a mass-to-charge ratio of a product ion, the MRM transition being one of measurement conditions of the MRM measurement, is:
   646.05/763.95, 646.05/410.25 or 646.05/615.35 for the peptide AD1008,
   538.95/534.25, 538.95/484.75 or 538.95/541.25 for the peptide AD1025,
   520.75/449.70, 520.75/401.20 or 520.75/801.40 for the peptide AD1042,
   856.90/720.25, 856.90/993.55 or 856.90/555.35 for the peptide AD1046,
   776.00/1061.50, 776.00/1036.45 or 776.00/1150.50 for the peptide AD1048,
   512.30/516.30, 512.30/629.35 or 512.30/453.75 for the peptide AD1049,
   518.25/642.85, 518.25/586.30 or 776.90/796.40 for the peptide ADPEP109315,
   725.40/378.15, 725.40/917.45 or 725.40/873.95 for the peptide ADPEP421488,
   744.35/885.45, 744.35/757.40 or 496.60/472.25 for the peptide ADPEP1315,
   942.00/448.20, 628.35/448.20 or 942.00/547.25 for the peptide ADPEP12neu,
   781.35/781.40, 521.25/533.30 or 781.35/1072.50 for the peptide ADPEP1396,
   638.80/1072.50, 638.80/862.40 or 638.80/747.35 for the peptide ADPEP1039,
   478.25/452.25, 478.25/509.75 or 478.25/351.20 for the peptide ADPEP1250, and
   458.25/331.20, 458.25/602.35 or 458.25/701.40 for the peptide AD1089.

The present invention has been made by finding an MRM transition, which is a combination of a mass-to-charge ratio of a precursor ion associated with each peptide and a mass-to-charge ratio of a product ion, as an optimum measurement condition when the above 14 peptides known as cognitive dysfunction disease biomarkers are quantified by performing MRM measurements using a mass spectrometer capable of MS/MS measurement. That is, by setting the transition in the MRM measurement as described above for each of the 14 peptides, each of the 14 peptides contained in a biological sample can be detected specifically and highly sensitively.

Also, the present invention provides a mass spectrometer that determines a content of a cognitive dysfunction disease biomarker based on a result of mass spectrometry of the cognitive dysfunction disease biomarker contained in a biological sample, the mass spectrometer including:
a mass analysis unit capable of an MS/MS measurement;
a measurement condition storage unit that stores MRM transitions of 14 peptides having the above-mentioned SEQ ID NOs 1 to 14 as measurement conditions including MRM transitions, each of the MRM transitions being a combination of a mass-to-charge ratio of a precursor ion and a mass-to-charge ratio of a product ion when the mass analysis unit performs an MRM measurement; and
a measurement execution unit that causes the mass analysis unit to perform MRM measurements of the 14 peptides using the measurement conditions stored in the measurement condition storage unit.

In the above configuration, the mass analysis unit is preferably a triple quadrupole mass analysis unit, and in that case, the measurement condition storage unit may store, in addition to the MRM transitions of 14 peptides, collision energy, the voltage value to be applied to each rod electrode of a front quadrupole mass filter and a rear quadrupole mass filter and the like. Here, the collision energy and the voltage value to be applied to each rod electrode of the front quadrupole mass filter and the rear quadrupole mass filter may be determined by the user who performs the measurement using the present apparatus by performing preliminary experiments or the like or may be determined by the manufacturer itself that manufactures the apparatus by performing preliminary experiments or the like. Alternatively, the manufacturer may provide measurement conditions to the user in response to a request from the user.

According to the present invention, biomarkers of cognitive dysfunction disease in a biological sample can be detected and quantified in a short time specifically and highly sensitively, so that an early diagnosis of cognitive dysfunction diseases can be made.

### BRIEF DESCRIPTION OF DRAWINGS

Fig. 1 is a schematic block diagram of an embodiment of a liquid chromatograph mass spectrometer used for carrying out a method for quantifying a cognitive dysfunction disease biomarker according to the present invention.
Fig. 2 is a diagram showing MRM transitions of 14 biomarker peptides of cognitive dysfunction diseases.
Fig. 3 is a diagram illustrating a procedure of preprocessing.
Fig. 4 is a diagram showing a gradient profile of analysis in a liquid chromatograph unit.
Fig. 5 is an MRM chromatogram obtained as a result of measuring a certain biological sample using the liquid chromatograph mass spectrometer according to the present embodiment.

### DESCRIPTION OF EMBODIMENTS

Hereinafter, an embodiment of a method for quantifying a cognitive dysfunction disease biomarker according to the present invention will be described with reference to the drawings.

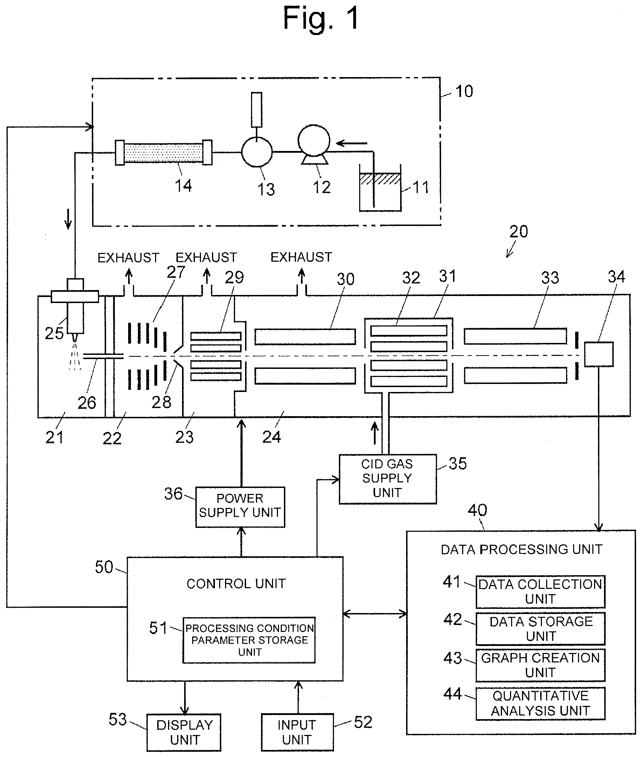

Fig. 1 is a schematic block diagram of a liquid chromatograph mass spectrometer (LC-MS/MS) used for carrying out the method for quantifying a cognitive dysfunction disease biomarker according to the present invention.

The liquid chromatograph mass spectrometer includes a liquid chromatograph unit (LC unit) 10 and a mass analysis unit (MS/MS unit) 20.

The LC unit 10 includes a mobile phase vessel 11 in which a mobile phase is stored, a pump 12 that sucks the mobile phase to feed the mobile phase at a constant flow rate, an injector 13 that injects a predetermined amount of sample prepared in advance into the mobile phase, and a column 14 that separates various compounds contained in the sample in a time direction. When so-called gradient separation is performed in which the LC unit 10 separates compounds contained in a sample while continuously changing the composition of a plurality of mobile phases, two or more mobile phase vessels 11 and two or more pumps 12 are provided.

The MS/MS unit 20 is constituted of a tandem quadrupole mass spectrometer and has a configuration of a multi-stage differential evacuation system including first and second intermediate vacuum chambers 22 and 23 having a degree of vacuum stepwisely increased between an ionization chamber 21 which is at atmospheric pressure and a high vacuum analysis chamber 24 evacuated by a high-performance vacuum pump. The ionization chamber 21 is provided with an electrospray ionization probe 25 for atomization while applying electric charge to a sample solution, and the ionization chamber 21 and the first intermediate vacuum chamber 22 at the next stage are communicated through a heating capillary 26 with a small diameter. The first intermediate vacuum chamber 22 and the second intermediate vacuum chamber 23 are separated by a skimmer 28 having a small hole at the top, and ion guides 27 and 29 for transporting ions to the latter stage while converging ions are installed in the first intermediate vacuum chamber 22 and the second intermediate vacuum chamber 23, respectively. In the analysis chamber 24, a front quadrupole mass filter 30 that separates ions in accordance with a mass-to-charge ratio while facing a collision cell 31 in which a multipole ion guide 32 is installed, a rear quadrupole mass filter 33 for similarly separating ions in accordance with a mass-to-charge ratio, and further an ion detector 34 are installed. A CID gas supply unit 35 supplies a CID gas such as argon or nitrogen into the collision cell 31. In addition, a power supply unit 36 applies predetermined voltages to the electrospray ionization probe 25, the ion guides 27, 29, 32, the quadrupole mass filters 30, 33, and the like.

A data processing unit 40 includes a data collection unit 41, a data storage unit 42, a graph creation unit 43, a quantitative analysis unit 44, and the like as functional blocks. A control unit 50 to which an input unit 52 and a display unit 53 are attached includes a processing condition parameter storage unit 51 and controls, based on processing condition parameters stored in advance in the storage unit 51, the operation of each unit such as the pump 12 and the injector 13 of the LC unit 10 and the power supply unit 36 and the CID gas supply unit 35 of the MS/MS unit 20. In the present embodiment, the control unit 50 functions as a measurement execution unit of the present invention, and the processing condition parameter storage unit 51 functions as a measurement condition mechanism unit. At least portion of the functions of the control unit 50 and the data processing unit 40 can be implemented by using a personal computer as a hardware resource and executing dedicated control and processing software installed in advance on the computer.

The basic MS/MS measurement operation in the LC-MS/MS according to the present embodiment is as follows.

First, the pump 12 sucks a mobile phase from the mobile phase vessel 11 and feeds the mobile phase to the column 14 at a constant flow rate. When a constant amount of sample liquid is introduced into the mobile phase from the injector 13, the sample is introduced into the column 14 in the flow of the mobile phase, and various compounds in the sample are separated in the time direction while passing through the column 14 and eluted from the outlet of the column 14 before being introduced into the MS/MS unit 20.

When the eluate from the column 14 reaches the electrospray ionization probe 25 in the MS/MS unit 20, the eluate is atomized while being charged at the tip of the probe 25. Charged droplets formed by atomization are fragmented while being split caused by the action of electrostatic force due to the applied charge, and in the process the solvent evaporates and ions derived from the compound pop out.

Ions thus generated are sent to the first intermediate vacuum chamber 22 through the heating capillary 26 and are converged by the ion guide 27 before being sent to the second intermediate vacuum chamber 23 via the small hole at the top of the skimmer 28. Then, the ions derived from the compound are converged by the ion guide 29 and sent to the analysis chamber 24 before being introduced into the space in the longitudinal direction of the front quadrupole mass filter 30. It should be noted that the ionization method is not limited to the electrospray ionization method and it is a matter of course that an atmospheric pressure chemical ionization method, an atmospheric pressure photoionization method or the like may be used.

When an MS/MS analysis is performed in the MS/MS unit 20, each rod electrode of the front quadrupole mass filter 30 and the rear quadrupole mass filter 33 is supplied with a predetermined voltage (voltage in which a high-frequency voltage and a direct-current voltage are superimposed) from the power supply unit 36, and a CID gas is continuously or intermittently supplied from the CID gas supply unit 35 into the collision cell 31. Accordingly, among various ions sent to the front quadrupole mass filter 30, only ions having a specific mass-to-charge ratio corresponding to the voltage applied to each rod electrode of the front quadrupole mass filter 30 pass through the filter 30 and are introduced into the collision cell 31 as precursor ions. In the collision cell 31, the precursor ions collide with the CID gas and dissociated, generating various product ions. When the generated various product ions are introduced into the rear quadrupole mass filter 33, only product ions having a specific mass-to-charge ratio corresponding to the voltage applied to each rod electrode of the rear quadrupole mass filter 33 pass through the filter 33 and reach the ion detector 34 before being detected. A pulse count type detector can be used as the ion detector 34 and in such a case, pulse signals corresponding to the number of incident ions are output as detection signals.

A detection signal of the ion detector 34 is digitized and input into the data processing unit 40. The data processing unit 40 creates a mass chromatogram in the vicinity of the time at which the compound appears for each compound to be measured, based on data sequentially input. Then, in the mass chromatogram, a peak corresponding to the compound to be measured is detected to calculate the peak area, and the concentration value of the compound to be measured, that is, the quantitative value is calculated by referring to a calibration curve, a table or the like indicating the relationship between the peak area value stored in the quantitative analysis unit 44 and the concentration.

Also in the present embodiment, similarly to the general LC-MS/MS, multiple reaction monitoring (MRM) measurements, product ion scan measurements, precursor ion scan measurements, and neutral loss scan measurements are prepared as the mode of MS/MS measurements.

In the MRM measurement, MS/MS measurements are carried out by allowing only ions having a predetermined mass-to-charge ratio in each of the front quadrupole mass filter 30 and the rear quadrupole mass filter 33, so that specific product ions corresponding to specific precursor ions derived from the peptide to be measured are detected. In the MRM measurement, it is possible to carry out measurements in which a plurality of channels in each of which the mass-to-charge ratio (m/z) of precursor ions and the mass-to-charge ratio (m/z) of product ions are paired is set. In the quantitative analysis of a cognitive dysfunction disease biomarker described below, the MRM measurement is used as the MS/MS measurement.

Next, a procedure when a quantitative analysis of a cognitive dysfunction disease biomarker is performed using the LC-MS/MS in the present embodiment will be described. It is assumed here that 14 partial peptides shown in Table 1 are used as cognitive dysfunction disease biomarkers. These 14 partial peptides are all generated as partial peptides as a result of proteins, which are present in a complete state in the living body of a person not suffering from a cognitive dysfunction disease (hereinafter, referred to as "intact protein"), being digested in the living body of a cognitive dysfunction disease patient. Alternatively, these 14 partial peptides are translated and synthesized as partial peptides due to splicing abnormality or the like in translation and synthesis of proteins. Table 1 shows the identification numbers (ID) representing the 14 partial peptides, the intact protein name as the source of the peptides, and the amino acid sequences of the peptides.

**[Table 1]**

| No. | Peptide ID | Protein name | Amino acid sequence |
|---|---|---|---|
| 1 | AD 1008 | Complement C4-A | APLQPVTPLQLFEGRRN |
| | | Complement C4-A | |
| 2 | AD1025 | Transcription factor AP-2 γ | PGRQSQEGAGLPSHHG |
| | | Transcription factor AP-2 gamma | |
| 3 | AD1042 | Oxytocin receptor | AAPPGAEGNRT |
| | | Oxytocin receptor | |
| 4 | AD1046 | E3 ubiquitin ligase HERC2 | KLAELPAAAQPSAEDSD |
| | | E3 ubiquitin-protein ligase HERC2 | |
| 5 | AD1048 (ADPEP2036) | Prothrombin | TATSEYQTFFNPRTFGSGEAD |
| | | Prothrombin | |
| 6 | AD1049 | Complement C3 | APVIHQEMIGGLRN |
| | | Complement C3 | |
| 7 | ADPEP109315 | Tumor necrosis factor receptor superfamily member 16 | QTASGQALKGDGGLYS |
| | | Tumor necrosis factor receptor superfamily | |
| 8 | ADPEP421488 | Gelsolin | GLGLSYLSSHIANVERVPFD |
| | | Gelsolin | |
| 9 | ADPEP1315 | Neurexin-2-β precursor | RSGGNATLQVDSWP |
| | | Neurexin-2-beta precursor | |
| 10 | ADPEP12neu | Neurexin 1-β | NIAIVGDVRLVGEVPSSGT |
| | | Neurexin 1-β | |
| 11 | ADPEP1396 | Prothrombin precursor | TATSEYQTFFNPR |
| | | Prothrombin precursor | |
| 12 | ADPEP1039 | Prothrombin precursor | GLDEDSDRAIEG |
| | | Prothrombin precursor | |
| 13 | ADPEP1250 | Prothrombin precursor | GLDEDSDRAIEGR |
| | | Prothrombin precursor | |
| 14 | AD 1089 | Protocadherin γ A-10 | GVSGSHFVGVDGVR |
| | | Protocadherin gamma A-10 | |

### <1. Measurement Condition Settings>

As shown in Table 1, the amino acid sequence of each of the 14 peptides is known, and by conducting a preliminary experiment or by using a general compound database, measurement conditions suitable for quantitative analysis using the LC-MS/MS in the present embodiment can be determined. As such measurement conditions, each value of the retention time of each peptide under predetermined separation conditions in the LC unit 10, a combination of the mass-to-charge ratio of precursor ions and the mass-to-charge ratio of product ions derived from each peptide, which is a condition for performing MRM measurement in the MS/MS unit 20 (hereinafter, referred to as a "MRM transition"), the collision energy, the voltage (Q1 pre-rod bias voltage) applied to each rod electrode of the front quadrupole mass filter 30, and the voltage (Q3 pre-rod bias voltage) applied to each rod electrode of the rear quadrupole mass filter 33 can be cited.

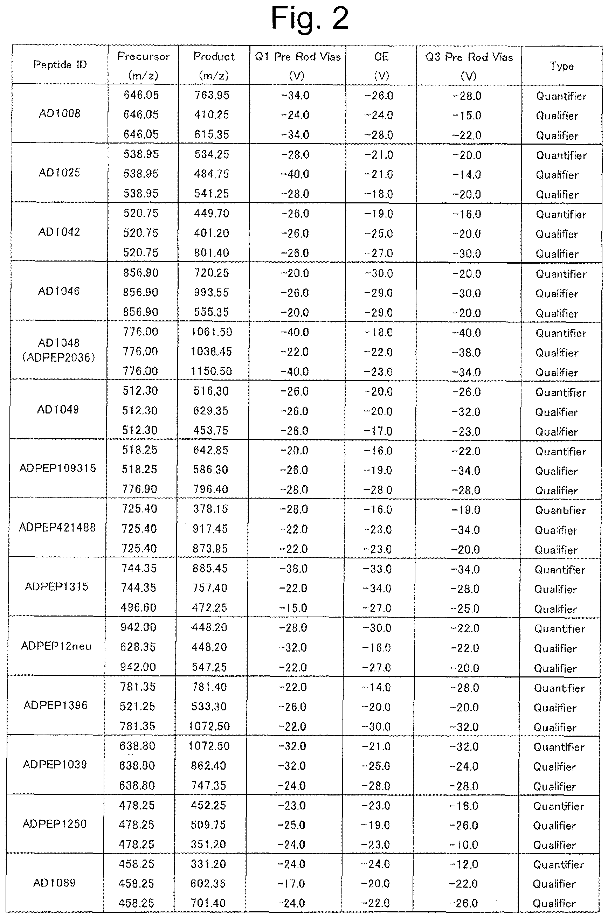

In the present embodiment, in order to be capable of determining the content of each of the 14 peptides in a biological sample, an appropriate MRM transition, the collision energy value, the Q1 pre-rod bias voltage value, and the Q3 pre-rod bias voltage value are determined in advance for each of the 14 peptides, and these MRM parameters are stored in the processing condition parameter storage unit 51. Fig. 2 shows MRM parameters for each peptide stored in the processing condition parameter storage unit 51.

Next, description will be given from the preparation of a biological sample to measurement processing in the LC unit 10 and the MS/MS unit 20.

### <2. Preparation of Reagents, Tools, and the like>

First, the following reagents, tools and the like are prepared.
(1) Standard solution for calibration curve (STD solution): A reagent containing a predetermined amount of peptide, more specifically a predetermined amount of one or a plurality of peptides selected from 14 peptides listed in Table 1, the peptide including a portion of the amino acid sequence of an intact protein (prothrombin, gelsolin, complement C4A, complement C3) that serves as a biomarker of cognitive dysfunction disease. The STD solution may be a standard serum containing a predetermined amount of one or a plurality of peptides selected from the 14 peptides. By stepwisely diluting the STD solution using a predetermined diluent, each calibration point solution (for example, solutions for six calibration points) on the calibration curve is prepared.
(2) Standard solution for quality control (QC solution): A solution prepared by mixing a solution for calibration point prepared from the STD solution. For example, in the case of six calibration points, a solution prepared by mixing the solution of calibration point 1 and the solution of calibration point 2, a solution prepared by mixing the solution of calibration point 3 and the solution of calibration point 4, and a solution prepared by mixing the solution of calibration point 5 and the solution of calibration point 6 are each used as QC solutions.
(3) Stable isotope reagent: A reagent containing a predetermined amount of the 14 peptides in which a portion of atoms is substituted with stable isotopes.
(4) Solvent A: A water-soluble organic solvent containing 0.1% to 1% trifluoroacetic acid (TFA) and 5% to 50% acetonitrile (ACN)
(5) Solvent B: 50% to 100% methanol
(6) Solvent C: An aqueous solution containing 0.1% to 1% TFA
(7) Solvent D: A water-soluble organic solvent containing 0.1% to 1% TFA and 5% to 50% methanol
(8) Aluminum seal
(9) A 96-hole (well) sample plate
(10) A plate mat for evaporation prevention

### <3. Preparation of Biological Sample (Serum)>

Blood is collected from the subject and left for a predetermined period of time, followed by centrifugation before the supernatant (serum) is collected.

### <4. Preparation of Sample>

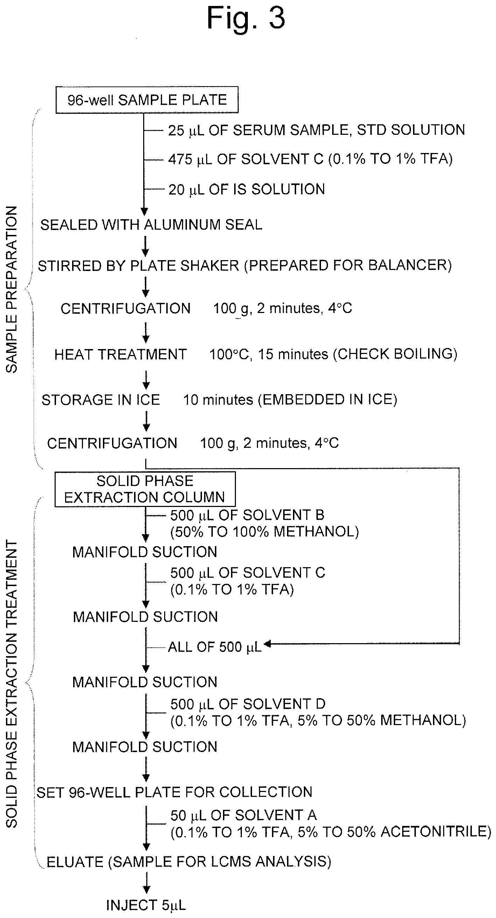

Preprocessing is performed according to the procedure shown in Fig. 3.

First, 25 µL of biological sample serum is injected into each well of the 96-well sample plate. In addition, 25 µL of the STD solution or QC solution is injected into wells different from the wells into which serum has been injected.

Next, 475 µL of the solvent C (an aqueous solution containing 0.1% to 1% TFA) is added to each well of the sample plate into which the serum, the STD solution, or the QC solution has been injected and further, 20 µL of the stable isotope reagent is added. Accordingly, 520 µL of liquid (mixed liquid) has been injected into each well.

Subsequently, the upper surface of the sample plate is sealed with an aluminum seal and the sample plate is stirred by a plate shaker. Then, the sample is subjected to the first centrifugation (100 g, 2 minutes, 4°C) by a centrifuge corresponding to the sample plate, heat treatment (100°C, 15 minutes), storage in ice (10 minutes), and the second centrifugation (100 g, 2 minutes, 4°C).

### <5. Treatment by Solid Phase Extraction Column (Solid Phase Extraction Plate)>

First, the solvent B (500 µL of 50% to 100% methanol) is added to each well of a solid phase extraction column (trade name: Oasis HLB µElution Plate, manufactured by Water Inc., hereinafter, referred to as the "solid phase extraction plate") corresponding to the 96-well plate to remove insoluble components, followed by adding 500 µL of the solvent C (aqueous solution containing 0.1% to 1% TFA) to adjust the solid phase extraction plate.

Next, 500 µL of the supernatant in each well of the sample plate after the second centrifugation of the pretreatment is added to the corresponding well of the solid phase extraction plate. Subsequently, 500 µL of the solvent D (water-soluble organic solvent containing [0.1% to 1% TFA] + [5% to 50% methanol]) is added into each well of the solid phase extraction plate to clean and discharge matrix components non-specifically adsorbed onto the solid phase extraction plate. Then, 50 µL of the solvent A (water-soluble organic solvent containing [0.1% to 1% TFA] + [5% to 50% ACN]) is added to each well of the solid phase extraction plate to elute peptide adsorbed onto the solid phase extraction plate into each well of the 96-well sample plate for mass spectrometry. The liquid contained in each well of the 96-well sample plate obtained as described above becomes a sample solution for mass spectrometry.

### <6. Measurement by Chromatograph Mass Spectrometer>

The 96-well sample plate containing sample solutions for mass spectrometry obtained by treatment with the solid phase extraction column was set onto a sample mounting portion of the injector 13 of the LC-MS/MS described above to perform chromatographic mass spectrometry . Specific apparatus names of the LC unit 10 and the MS/MS unit 20, treatment conditions and the like in each unit, and analysis results thereof are shown below.

### <6.1 LC unit>

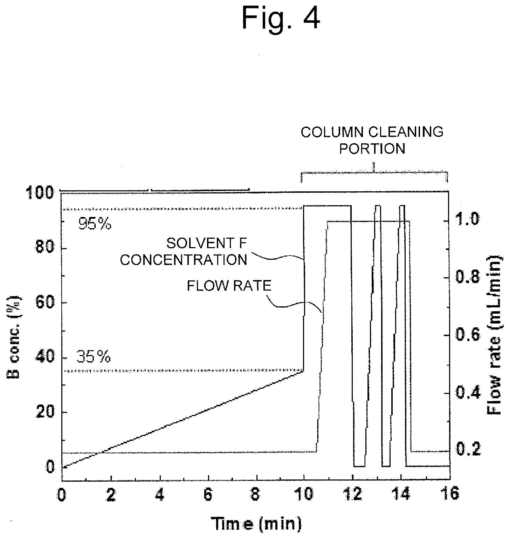

- Apparatus name: A high-performance liquid chromatograph apparatus (trade name: Nexera XR (manufactured by Shimadzu Corporation)) capable of gradient analysis was used.
- Column: A reversed-phase column for analysis of peptides and proteins (trade name: Aeris (registered trademark) peptide (Phenomenex Inc.), column size: 2.1 × 50 mm, 2.6 µm (MAX Pressure: 1000 bar (100 MPa)) was used.
- Analysis method: A gradient analysis was adopted in which the sample solution for mass spectrometry was temporally separated while temporally changing the mixing ratio of solvent E and solvent F below. Fig. 4 shows a gradient profile.
   Solvent E: Water: ACN: formic acid = 98: 2: 0.1
   Solvent F: Water: ACN: formic acid = 10: 90: 0.1
   Heating temperature; 55°C
   Sampler temperature; 5°C

### <6.2 MS/MS unit>

- Apparatus name: A triple quadrupole mass spectrometer (trade name: LCMS-8060 (manufactured by Shimadzu Corporation)), which is a mass spectrometer capable of MRM measurement, was used. The setting values of each unit at the time of analysis are as follows:
   CID gas pressure: 270kPa
   Interface voltage: 3kV
   Nebulizer gas flow rate: 3L/min
   Heating gas flow rate: 10L/min
   Interface temperature: 300°C
   DL temperature: 250°C
   Heat block temperature: 400°C
   Drying gas flow rate: 10L/min

The MRM measurement in the MS/MS unit 20 was performed by using the MRM parameters (see Fig. 2) of each peptide stored in advance in the processing condition parameter storage unit 51.

### <6.3 Measurement results>

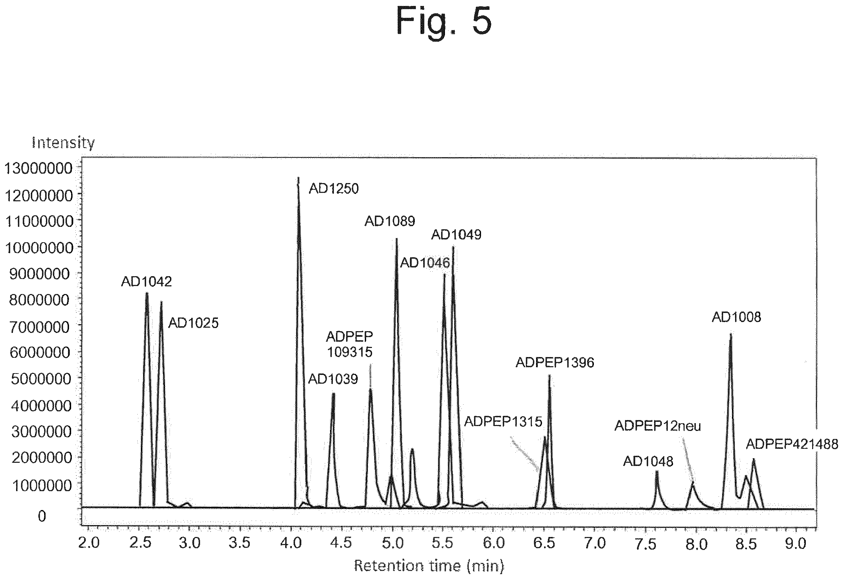

Fig. 5 is a base peak chromatogram created based on the ionic strength of the obtained 14 peptides as a result of conducting liquid chromatograph mass spectrometry on a biological sample (serum) collected from a certain subject. The symbols shown in Fig. 5 correspond to those shown in Table 1. As shown in Fig. 5, peaks derived from the 14 peptides were respectively observed.

The above embodiment is merely an example of the present invention, and for example, a gas chromatograph may be used instead of the liquid chromatograph.

In the above embodiment, MRM parameters for each peptide are stored in advance in the processing condition parameter storage unit 51 for all of the 14 peptides, but MRM parameters for each peptide may be stored in the processing condition parameter storage unit 51 for one or a plurality of peptides selected from the 14 peptides.

In addition, the tools and apparatuses used in the above embodiment are merely examples, and the present invention is not limited thereto. For example, a solid phase extraction plate may be constituted by using a C18 column generally used as a reversed phase column of liquid chromatography.

In addition, it is obvious that even if appropriate modifications, amendments, or additions are made within the scope of the spirit of the present invention, these modifications, amendments, or additions are included within the scope of claims of the present application.

### REFERENCE SIGNS LIST

- 10: Liquid Chromatograph Unit
- 14: Column
- 20: MS/MS Unit
- 30: Quadrupole Mass Filter
- 30: Front Quadrupole Mass Filter
- 31: Collision Cell
- 32: Ion Guide
- 34: Ion Detector
- 35: Cid Gas Supply Unit
- 36: Power Supply Unit
- 40: Data Processing Unit
- 41: Data Collection Unit
- 42: Data Storage Unit
- 43: Graph Creation Unit
- 44: Quantitative Analysis Unit
- 50: Control Unit
- 52: Input Unit
- 53: Display Unit

## Claims

1. A method for quantifying a cognitive dysfunction disease biomarker based on a result obtained by performing a multiple reaction monitoring (MRM) measurement using a mass spectrometer capable of MS/MS measurement of the cognitive dysfunction disease biomarker contained in a biological sample,
wherein
the cognitive dysfunction disease biomarker includes at least one peptide selected from the group consisting of:
a complement C4-A-derived peptide AD1008 composed of an amino acid sequence represented by SEQ ID NO: 1,
a transcription factor AP-2γ-derived peptide AD1025 composed of an amino acid sequence represented by SEQ ID NO: 2,
an oxytocin receptor-derived peptide AD1042 composed of an amino acid sequence represented by SEQ ID NO: 3,
an E3 ubiquitin ligase HERC-derived peptide AD1046 composed of an amino acid sequence represented by SEQ ID NO: 4,
a prothrombin-derived peptide AD1048 composed of an amino acid sequence represented by SEQ ID NO: 5,
a complement C4-derived peptide AD1049 composed of an amino acid sequence represented by SEQ ID NO: 6,
a tumor necrosis factor receptor superfamily member 16-derived peptide ADPEP109315 composed of an amino acid sequence represented by SEQ ID NO: 7,
a gelsolin-derived peptide ADPEP421488 composed of an amino acid sequence represented by SEQ ID NO: 8,
a neurexin-2-β precursor-derived peptide ADPEP 1315 composed of an amino acid sequence represented by SEQ ID NO: 9,
a neurexin-1-β-derived peptide ADPEP12neu composed of an amino acid sequence represented by SEQ ID NO: 10,
a prothrombin precursor-derived peptide ADPEP1396 composed of an amino acid sequence represented by SEQ ID NO: 11,
a prothrombin precursor-derived peptide ADPEP1039 composed of an amino acid sequence represented by SEQ ID NO: 12,
a prothrombin precursor-derived peptide ADPEP1250 composed of an amino acid sequence represented by SEQ ID NO: 13, and
a protocadherin γ-derived peptide AD1089 composed of an amino acid sequence represented by SEQ ID NO: 14, and
an MRM transition which is a combination of a mass-to-charge ratio of a precursor ion and a mass-to-charge ratio of a product ion, the MRM transition being one of measurement conditions of the MRM measurement, is:
646.05/763.95, 646.05/410.25 or 646.05/615.35 for the peptide AD1008,
538.95/534.25, 538.95/484.75 or 538.95/541.25 for the peptide AD1025,
520.75/449.70, 520.75/401.20 or 520.75/801.40 for the peptide AD1042,
856.90/720.25, 856.90/993.55 or 856.90/555.35 for the peptide AD1046,
776.00/1061.50, 776.00/1036.45 or 776.00/1150.50 for the peptide AD1048,
512.30/516.30, 512.30/629.35 or 512.30/453.75 for the peptide AD1049,
518.25/642.85, 518.25/586.30 or 776.90/796.40 for the peptide ADPEP109315,
725.40/378.15, 725.40/917.45 or 725.40/873.95 for the peptide ADPEP421488,
744.35/885.45, 744.35/757.40 or 496.60/472.25 for the peptide ADPEP1315,
942.00/448.20, 628.35/448.20 or 942.00/547.25 for the peptide ADPEP12neu,
781.35/781.40, 521.25/533.30 or 781.35/1072.50 for the peptide ADPEP1396,
638.80/1072.50, 638.80/862.40 or 638.80/747.35 for the peptide ADPEP1039,
478.25/452.25, 478.25/509.75 or 478.25/351.20 for the peptide ADPEP1250, and
458.25/331.20, 458.25/602.35 or 458.25/701.40 for the peptide AD1089.

2. The method for quantifying a cognitive dysfunction disease biomarker using a mass spectrometer according to claim 1, wherein the mass spectrometer is a triple quadrupole mass spectrometer.

3. A mass spectrometer that determines a content of a cognitive dysfunction disease biomarker based on a result of mass spectrometry of the cognitive dysfunction disease biomarker contained in a biological sample, the mass spectrometer comprising:
a mass analysis unit capable of an MS/MS measurement;
a measurement condition storage unit that stores MRM transitions of 14 peptides as measurement conditions including MRM transitions, each of the MRM transitions being a combination of a mass-to-charge ratio of a precursor ion and a mass-to-charge ratio of a product ion when the mass analysis unit performs an MRM measurement; and
a measurement execution unit that causes the mass analysis unit to perform MRM measurements of the 14 peptides using the measurement conditions stored in the measurement condition storage unit,
wherein
the 14 peptides are:
a complement C4-A-derived peptide AD1008 composed of an amino acid sequence represented by SEQ ID NO: 1,
a transcription factor AP-2γ-derived peptide AD1025 composed of an amino acid sequence represented by SEQ ID NO: 2,
an oxytocin receptor-derived peptide AD1042 composed of an amino acid sequence represented by SEQ ID NO: 3,
an E3 ubiquitin ligase HERC-derived peptide AD1046 composed of an amino acid sequence represented by SEQ ID NO: 4,
a prothrombin-derived peptide AD1048 composed of an amino acid sequence represented by SEQ ID NO: 5,
a complement C4-derived peptide AD1049 composed of an amino acid sequence represented by SEQ ID NO: 6,
a tumor necrosis factor receptor superfamily member 16-derived peptide ADPEP109315 composed of an amino acid sequence represented by SEQ ID NO: 7,
a gelsolin-derived peptide ADPEP421488 composed of an amino acid sequence represented by SEQ ID NO: 8,
a neurexin-2-β precursor-derived peptide ADPEP 1315 composed of an amino acid sequence represented by SEQ ID NO: 9,
a neurexin-1-β-derived peptide ADPEP12neu composed of an amino acid sequence represented by SEQ ID NO: 10,
a prothrombin precursor-derived peptide ADPEP1396 composed of an amino acid sequence represented by SEQ ID NO: 11,
a prothrombin precursor-derived peptide ADPEP1039 composed of an amino acid sequence represented by SEQ ID NO: 12,
a prothrombin precursor-derived peptide ADPEP1250 composed of an amino acid sequence represented by SEQ ID NO: 13, and
a protocadherin γ-derived peptide AD1089 composed of an amino acid sequence represented by SEQ ID NO: 14, and
the MRM transitions of the 14 peptides are:
646.05/763.95, 646.05/410.25 or 646.05/615.35 for the peptide AD1008,
538.95/534.25, 538.95/484.75 or 538.95/541.25 for the peptide AD1025,
520.75/449.70, 520.75/401.20 or 520.75/801.40 for the peptide AD1042,
856.90/720.25, 856.90/993.55 or 856.90/555.35 for the peptide AD1046,
776.00/1061.50, 776.00/1036.45, or 776.00/1150.50 for the peptide AD1048,
512.30/516.30, 512.30/629.35 or 512.30/453.75 for the peptide AD1049,
518.25/642.85, 518.25/586.30 or 776.90/796.40 for the peptide ADPEP109315,
725.40/378.15, 725.40/917.45, or 725.40/873.95 for the peptide ADPEP421488,
744.35/885.45, 744.35/757.40 or 496.60/472.25 for the peptide ADPEP1315,
942.00/448.20, 628.35/448.20 or 942.00/547.25 for the peptide ADPEP12neu,
781.35/781.40, 521.25/533.30 or 781.35/1072.50 for the peptide ADPEP1396,
638.80/1072.50, 638.80/862.40 or 638.80/747.35 for the peptide ADPEP1039,
478.25/452.25, 478.25/509.75 or 478.25/351.20 for the peptide ADPEP1250, and
458.25/331.20, 458.25/602.35 or 458.25/701.40 for the peptide AD1089.

4. The mass spectrometer according to claim 3, wherein
the mass analysis unit is a triple quadrupole mass analysis unit, and
the measurement conditions stored in the measurement condition storage unit further include one or a plurality of values selected from collision energy, a voltage value to be applied to each rod electrode of a front quadrupole mass filter, and a voltage value to be applied to each rod electrode of a rear quadrupole mass filter.
